# EUROPEAN PATENT SPECIFICATION

(11) **EP 1 965 201 B1**
(45) Date of publication and mention of the grant of the patent: **22.04.2009**
(21) Application number: 07004177.7
(22) Date of filing: 28.02.2007
(51) Int. Cl.: G01N 27/327, C12Q 1/00, G01N 33/92

(54) **Sensing member for detecting total cholesterol of blood sample**
Messelement für den Nachweis des Gesamtcholesterins in einer Blutprobe
Elément capteur pour détecter le cholestérol total dans un échantillon de sang

(43) Date of publication of application: 03.09.2008
(60) Divisional application: 08170135.1
(73) Proprietor: General Life Biotechnology Co., Ltd., Jhongsiao E. Rd. Taipei City 106 (TW)
(72) Inventor: Su, Chein-Shyong, Taipei City 106 (TW); Hung, Miao-Ling, Taipei City 106 (TW); Jing, Ning-Jun, Taipei City 106 (TW); Wu, Tai-Guang, Taipei City 106 (TW)
(74) Representative: Epping - Hermann - Fischer

(56) References cited:
- EP-A1- 1 223 425
- US-A- 5 356 786
- US-A- 6 033 866
- DATABASE WPI Week 200462 Derwent Publications Ltd., London, GB; AN 2004-637906 XP002440331 & JP 2004 245736 A (TECHNO MEDICA KK) 2 September 2004 (2004-09-02)

## Description

### BACKGROUND OF THE INVENTION

### 1. Field of the Invention:

The present invention relates generally to a sensing member for detecting total cholesterol of blood sample, and more particularly to a sensing member that detects total cholesterol of blood sample through an electrochemical method.

### 2. Description of Related Art:

Total cholesterol level in blood constitutes an important part of a lipid profile, which is generally a primary focus in preventing and/or curing diseases such as arteriosclerosis and cardiovascular diseases. If cholesterol level in blood can be detected conveniently in our daily life so as to timely control the cholesterol in blood within a standard value, probability of occurrence of arteriosclerosis and cardiovascular diseases will be effectively reduced.

Currently, blood cholesterol level can be detected through a biochemical molecular level detection method, which uses spectrophotometric instrumentation combined with assay of chromogenic reaction of reactive composition at specified wavelength such as light absorption. However, a spectrophotometric method is considered to give a qualitative or semi-quantitative detection result and such instrumentation must be used in specified locations such as in a laboratory, which is rather inconvenient. Meanwhile, this method has drawback of instability in determining biochemical molecules of low concentration.

An electrochemical detecting method is a rapid and reliable approach, which uses a conducting electrode to detect electrical current positively proportional to the concentration of analyte of the sample transported by electrons generated in a biochemical reaction through an enzyme. As an electrode detecting sheet is easy to produce, has a low cost and can be conveniently transported, it has been widely applied in various kinds of biochemical detectors for detecting such as blood sugar, uric acid and cholesterol.

Generally, blood samples used in cholesterol level detection needs to be preprocessed through several steps such as centrifuging, depositing and separating such that materials such as corpuscles that may interfere the detecting result can be removed. However, as such preprocessing steps cannot be performed at home, a whole blood sample must be used in cholesterol level detection. US Patent No.5,695,947 discloses a biosensor used for detecting cholesterol level, wherein blood serum is used as a detecting sample. However, the patent does not disclose how the corpucles of the whole blood sample and other interfering materials will affect the detecting result.

US Patent No.6,033,866 discloses a biosensor which performs blood sugar detection based on an electrochemical assay principle. According to this patent, when a sample is dripped into a reactive pad located in an interlayer through an opening located on the reference electrode, corpuscles and interfering materials are separated from the sample through a separating film. However, cholesterol detection is different from blood sugar detection. Particularly, compared with adverse effect of interfering compositions on blood sugar detection, adverse effect of interfering compositions of a whole blood sample on the cholesterol detection can be much more serious. In addition, said patent does not disclose how position of the separating film will affect the detection result.

US Patent Publication No.2003/0183591 discloses a biosensor capable of separating corpuscles from a whole blood sample. When a whole blood sample enters into a filtering area, flowing rate of corpuscles is decreased according to the principle of horizontal chromatography, thus allowing the plasma to reach the electrode reaction area for detection. However, the horizontal chromatography needs a long sample separating time. Further, the flow rate of the corpuscles is difficult to be controlled, and it is .impossible to completely keep the corpuscles from entering into the reaction area. On the other hand, Japan Publication No.2004-245736 discloses a detecting sheet capable of using a whole blood sample to detect HDL cholesterol and triglycerol. However, the test sheet has a complex structure, and a corpuscle delay agent is needed. In addition, filtering of corpuscles and flowing of plasma depend on an additional pumping mechanism. Therefore, as much as several tens of microliter samples are needed and reactive time can reach several tens of minutes.

EP Patent Publication EP 1 223 425 discloses a biosensor for detecting blood cholesterol. If a sample solution is applied to the sample supply unit the filter removes the solid components from the sample and the remaining plasma flows through the filter and fills up a slit between base plate, spacer and cover. Thus, the plasma gets in contact with the reaction reagent system. If a voltage is applied to the working electrode an oxidation reaction occurs as soon as the obtained solution contacts the electrode.

There is a need to provide a sensor that can directly use whole blood sampled for detection and can rapidly and accurately obtain detection results.

### SUMMARY OF THE INVENTION

According to the above drawbacks, an objective of the present invention is to provide a sensing member capable of quickly detecting total cholesterol of blood samples.

Another objective of the present invention is to provide a sensing member capable of accurately detecting total cholesterol of blood samples.

A further objective of the present invention is to provide a sensing member capable of detecting total cholesterol of whole blood samples.

In order to attain the above and other objectives, the present invention discloses a sensing member for detecting total cholesterol of a blood sample, which comprises: a working electrode and a counter electrode respectively formed on a first substrate and a second substrate; a dielectric layer formed on one of the surfaces of the first and second substrates and exposing the electrode and end connector such that the end connector can be electrically connected to an electronic device used for applying electric voltage; a reactive pad disposed between the working electrode and the counter electrode and comprising an reactive reagent, wherein the reactive reagent comprises an electron transport material, an enzyme capable of reacting with the analyte and generating a current corresponding to the concentration, and a buffer solution; and a separating pad disposed between the first and second substrates, wherein the blood sample is passed through the separating pad such that interfering materials can be separated and removed from the blood sample and then the blood sample is laterally diffused to the reactive pad. By using the separating pad disposed between the first and second substrates, interfering materials of the blood sample can be removed from the blood sample. Thereafter, the blood sample is laterally diffused to the reactive pad for total cholesterol detection. Thus, the sensing member of the present invention can quickly and accurately detect total cholesterol of whole blood samples.

### BRIEF DESCRIPTION OF DRAWINGS

FIG. 1 is an exploded diagram of a sensing member for detecting total cholesterol of blood;
FIG.2 is another an exploded diagram of a sensing member for detecting total cholesterol of blood;
FIG.3 is an another exploded diagram of a sensing member for detecting total cholesterol of blood; and
FIG.4 is an exploded diagram of a sensing member for detecting total cholesterol of blood according to the present invention.

### DETAILED DESCRIPTION OF PREFERRED EMBODIMENTS

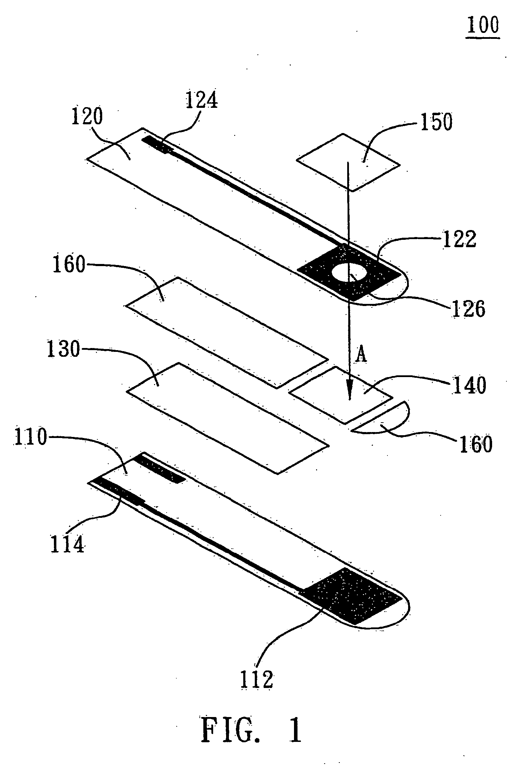

FIG 1 shows a sensing member (which does not fall within the scope of protection of the claims) for detecting total cholesterol of blood according to the present invention. As shown in FIG 1, the sensing member 100 comprises a first substrate 110, a second substrate 120, a dielectric layer 130, a reactive pad 1.40, and a separating pad 150. The substrates can be made of dielectric polymer, SiO₂, or AlO₂. Therein, the dielectric polymer can be, but not limited to PVC, polystyrene, polyester, polycarbonate, polyether, polyethylene, polypropylene and PET. In the present embodiment, PET sheet substrates are used as the first and second substrates 110, 120. Through a screen printing method, a working electrode 112 and metallic conducting lines 114 are formed on the first substrate 110, and a counter electrode 122 and metallic conducting lines 124 are formed on the second substrate 120. The second substrate 120 has an opening 126 formed at a position corresponding to the counter electrode 122 for allowing the blood sample to pass through. The working electrode and the counter electrode may be made of carbon electrode, graphite electrode, or metal particle. Preferably, the working electrode and the counter electrode are made of carbon electrode. The metallic conducting lines may be made of silver, gold or platinum. Preferably, the metallic conducting lines are made of silver.

A dielectric layer 130 is formed on surface of the first substrate 110 so as to cover the surface thereof. Therein, the working electrode 112 and the electrode end connector are exposed from the dielectric layer 130 such that the end connector can be electrically connected to an electronic device used for applying electric voltage. An adhesive layer 160 has an empty location formed corresponding to the working electrode 112 and the counter electrode 122. The reactive pad 140 comprising reactive reagent is disposed in the empty location of the adhesive layer 160 and then, the first substrate 110 with the working electrode 112 and the dielectric layer 130 and the second substrate 120 with the counter electrode 122 are attached together face by face, thereby disposing the reactive pad 140 comprising the reactive reagent between the working electrode 112 of the first substrate 110 and the counter electrode 122 of the second substrate 120.

The reactive reagent of the reactive pad 140 comprises an electron transport material, an enzyme capable of reacting with the analyte and generating a current corresponding to the concentration of the analyte, and a buffer solution. The electron transport material can be, but not limited to ferricyanate such as potassium ferricyanide, ferrocene, para-benzoquinone, phenazine methosulfate, Indophenol, TMB(3,3',5,5'-Tetramethylbenzidine) and β-naphthoquinone-4-potassium sulfonate. The enzyme capable of reacting with the analyte and generating a current corresponding to the concentration comprises cholesterol esterase, cholesterol oxidase and catalase. The buffer solution can be phosphate buffer solution, TRIS buffer solution or MES buffer solution. The reactive pad 140 can further comprise a surface active reagent such as Triton X-100 for improving solubility of ester cholesterol. Dripping the reactive reagent to the reactive pad 140 and roasting it 30 minutes at 40°C,a reactive pad 140 comprising the reactive reagent can be formed.

Subsequently, the separating pad 150 such as a glass fiber filter paper is disposed on the opening 126 of the second substrate 120. Thus, the sensing member 100 is formed. The separating pad 150 is used to remove interfering materials from the blood sample before the blood sample enters into the reactive pad 140 through the opening 126 in direction A as shown in FIG.1. Particularly, the separating pad of the present invention is capable of removing corpuscles and interfering materials from a whole blood sample, thus quickly and accurately obtaining detection results.

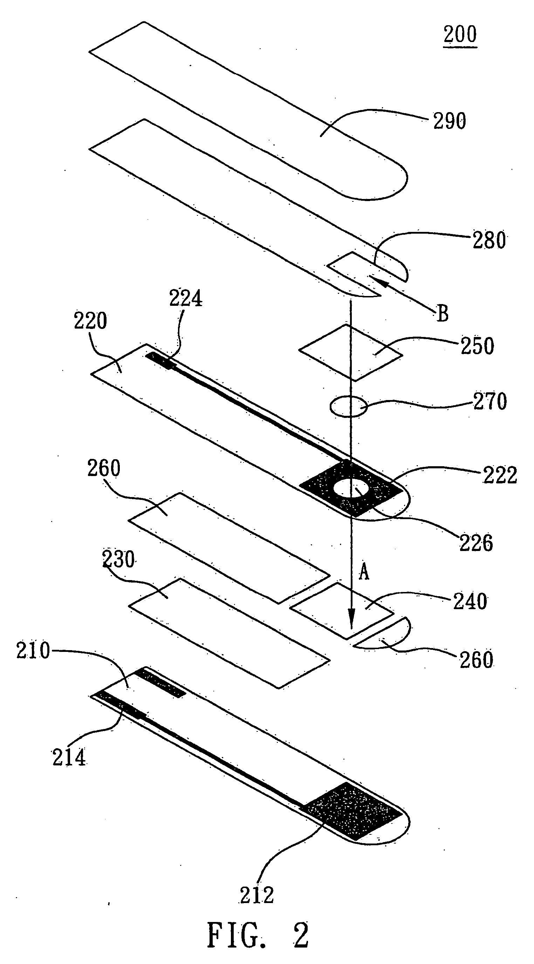

FIG.2 shows a sensing member (which does not fall under the scope of the claims) for detecting total cholesterol of blood. As shown in FIG.2, the sensing member 200 comprises a first substrate 210, a second substrate 220, a dielectric layer 230, a reactive pad 240, a separating pad 250, a flow conducting sheet 270, a capillary introducing area 280, and an upper covering sheet 290. Through a screen print method, a working electrode 212 and metallic conducting lines 214 are formed on the first substrate 210, and a counter electrode 222 and metallic conducting lines 224 are formed on the second substrate 220. The second substrate 220 has a circular opening 226 formed at a position corresponding to the counter electrode 222 for allowing the blood sample to pass through.

A dielectric layer 230 is formed on surface of the first substrate 210 so as to cover the surface thereof. Therein, the working electrode 212 and the electrode end connector are exposed from the dielectric layer 230 such that the end connector can be electrically connected to the electronic device used for applying electric voltage. An adhesive layer 260 has an empty location formed corresponding to the working electrode 212 and the counter electrode 222. The reactive pad 240 comprising reactive reagent is disposed on the empty location of the adhesive layer 260 and then, the first substrate 210 with the working electrode 212 and the dielectric layer 230 and the second substrate 220 with the counter electrode 222 are attached together face by face, thereby disposing the reactive pad 240 comprising the reactive reagent between the working electrode 212 of the first substrate 210 and the counter electrode 222 of the second substrate 220.

Subsequently, the flow conducting sheet 270 and the separating pad 250 are disposed in sequence on the circular opening 226 of the second substrate 220. The flow conducting sheet 270 substantially has a same outline as the circular opening 226, which conducts the blood sample from the separating pad 250 through the circular opening 226 into the reactive pad 240 in direction A as shown in FIG 2. The capillary introducing area 280 is disposed above the separating area 250 so as to smoothly conduct the blood sample into the separating pad 250 in direction B as shown in FIG.2. Finally, the upper covering sheet 290 covers the said structure, thus forming a sensing member.

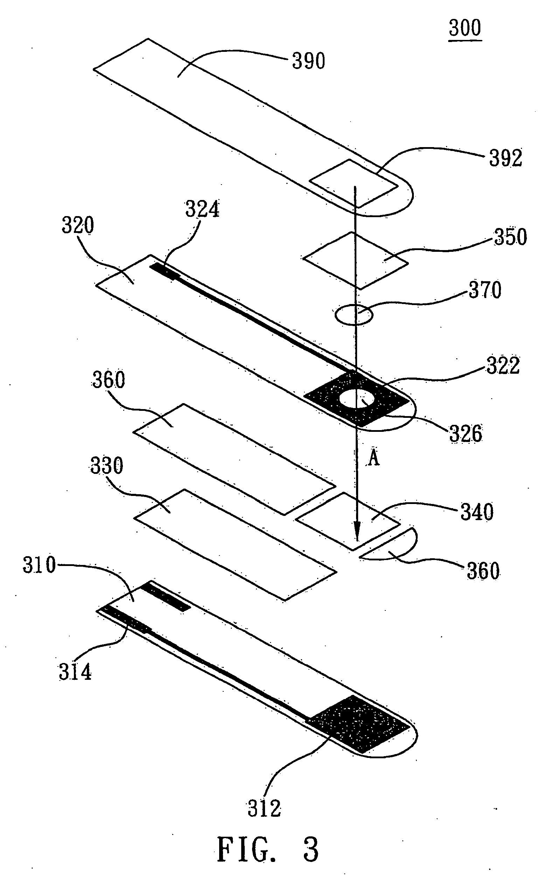

FIG.3 shows a sensing member (which does not fall within the scope of protection of the claims) for detecting total cholesterol of blood according to a third embodiment of the present invention. As shown in FIG.3, the sensing member 300 comprises a first substrate 310, a second substrate 320, a dielectric layer 330, a reactive pad 340, a separating pad 350, a flow conducting sheet 370, and an upper covering sheet 390. Through a screen print method, a working electrode 312 and metallic conducting lines 314 are formed on the first substrate 310, and a counter electrode 322 and metallic conducting lines 324 are formed on the second substrate 320. The second substrate 320 has a circular opening 326 formed at a position corresponding to the counter electrode 322 for allowing the blood sample to pass through.

A dielectric layer 330 is formed on surface of the first substrate 310 so as to cover the surface thereof. Therein, the working electrode 312 and the electrode end connector are exposed from the dielectric layer 330 such that the end connector can be electrically connected to the electronic device used for applying electric voltage. An adhesive layer 360 has an empty location formed corresponding to the working electrode 312 and the counter electrode 322. The reactive pad 340 comprising reactive reagent is disposed on the empty location of the adhesive layer 360 and then, the first substrate 310 with the working electrode 312 and the dielectric layer 330 and the second substrate 320 with the counter electrode 322 are attached together face by face, thereby disposing the reactive pad 340 comprising the reactive reagent between the working electrode 312 of the first substrate 310 and the counter electrode 322 of the second substrate 320.

Subsequently, the flow conducting sheet 370 and the separating pad 350 are disposed in sequence on the circular opening 326 of the second substrate 320, and then the upper covering sheet 390 is used to cover the said structure, thus forming a sensing member. Therein, the upper covering sheet 390 has an inlet 392 formed corresponding in position to the separating pad 350. The blood sample is injected into the sensing member through the inlet 392, then the blood sample flows through the separating pad 350, the flow conducting sheet 370 and the circular opening 326 and enters into the reactive pad 340 in direction A as shown in FIG.3.

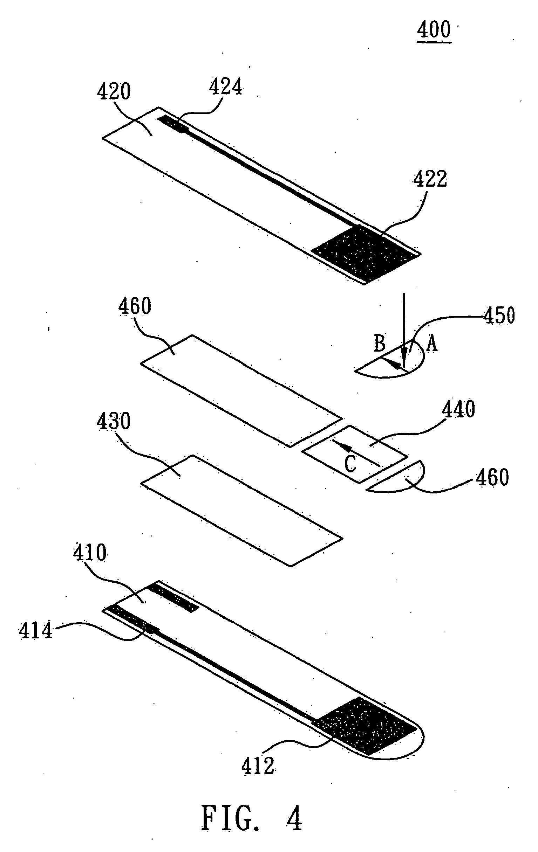

FIG.4 shows a sensing member for detecting total cholesterol of blood according to the present invention. As shown in FIG.4, the sensing member 400 comprises a first substrate 410, a second substrate 420, a dielectric layer 430, a reactive pad 440, and a separating pad 450. Through a screen print method, a working electrode 412 and metallic conducting lines 414 are formed on the first substrate 410, and a counter electrode 422 and metallic conducting lines 424 are formed on the second substrate 420. A dielectric layer 430 is formed on surface of the first substrate 410 so as to cover the surface of the first substrate 410. Therein, the working electrode 412 and the electrode end connector are exposed from the dielectric layer 430 such that the end connector can be electrically connected to the electronic device used for applying electric voltage. An adhesive layer 460 has an empty location formed corresponding to the working electrode 412 and the counter electrode 422. The reactive pad 440 comprising reactive reagent is disposed in the empty location of the adhesive layer 460 and then, the first substrate 410 with the working electrode 412 and the dielectric layer 430 and the second substrate 420 with the counter electrode 422 are attached together face by face, thereby disposing the reactive pad 440 comprising the reactive reagent between the working electrode 412 of the first substrate 410 and the counter electrode 422 of the second substrate 420.

The separating pad 450 is disposed between the second substrate 420 and the first substrate 410 at the injection end of blood sample. Preferably, the separating pad 450 is partially exposed so as to facilitate the injection of blood sample. As shown in FIG.4, the blood sample to be detected is injected into the sensing member 400 in direction A, then passes through the separating pad 450 in direction B such that interfering materials of the blood sample can be removed therefrom, and finally the blood sample is laterally diffused to the reactive pad 440 in direction C.

### Embodiment

### Embodiment 1-total cholesterol detection of blood plasma sample

The sensing member of Figure 3 is used to detect total cholesterol of a blood plasma sample. Apply voltage of -330mV on the metallic conducting lines of the working electrode, drip a blood plasma sample of 7µL into the inlet, after about 60 seconds of reaction time, read and record current intensity (µA) and total cholesterol concentration (mg/dL) in the sample marked by Kodak analyzer. Result is shown in Table 1.

**Table 1**

| | | | |
|---|---|---|---|
| Cholesterol concentration (mg/dL) | 121 | 198 | 275 |
| Current intensity (µA) | 1.1 | 2.2 | 4.0 |

### Embodiment 2-total cholesterol detection of whole blood sample

The sensing member of Figure 3 is used to detect total cholesterol of a whole blood sample. Apply voltage of -330mV on the metallic conducting lines of the working electrode, drip a whole blood of 10µL into the inlet, after about 60 seconds of reaction time, read and record current intensity (µA) and total cholesterol concentration (mg/dL) in the sample marked by Kodak analyzer. Result is shown in Table 2.

**Table 2**

| | | | | | |
|---|---|---|---|---|---|
| Cholesterol concentration (mg/dL) | 116 | 150 | 237 | 262 | 304 |
| Current intensity (µA) | 1.01 | 1.56 | 2.49 | 2.63 | 3.31 |

### Comparative example 1-without using the separating pad

The sensing member of Figure 3 is still used to detect total cholesterol of a whole blood sample. But the separating pad is not disposed in the sensing member according to this example. Apply voltage of -330mV on the metallic conducting lines of the working electrode, drip a whole blood of 10µL into the inlet, after about 60 seconds of reaction time, read and record current intensity (µA) and total cholesterol concentration (mg/dL) in the sample marked by Kodak analyzer. The result shows that there is no difference between current signals for whole blood samples respectively containing total cholesterol of 140 mg/dL and 300 mg/dL.

### Comparative example 2-changing position of the separating pad

The sensing member of Figure 3 is still used to detect total cholesterol of a whole blood sample. But the separating pad is disposed between the reactive pad and the second substrate. Apply voltage of -330mV on the metallic conducting lines of the working electrode, drip a whole blood of 10µL into the inlet, after about 60 seconds of reaction time, read and record current intensity (µA) and total cholesterol concentration (mg/dL) in the sample marked by Kodak analyzer. The result shows that there is no difference between current signals for whole blood samples respectively containing total cholesterol of 140 mg/dL and 300 mg/dL.

According to the result in Table 2, current intensity is linearly proportional to total cholesterol of the blood sample. However, in the comparative examples 1 and 2, if the separating pad is not disposed in the sensing member or the position of the separating pad is changed, whole blood samples having total cholesterol of 140 mg/dL to 300 mg/dL cannot be accurately detected. Therefore, the sensing member with the separating pad disposed at specified position can be used to quickly and accurately detect content of total cholesterol of whole blood samples, especially content of total cholesterol of whole blood samples.

## Claims

1. A sensing member for detecting total cholesterol of a blood sample, comprising:
a working electrode and a counter electrode respectively formed on a first substrate and a second substrate;
a dielectric layer formed on one of the surfaces of the first and second substrates and exposing the electrode and end connector such that the end connector can be electrically connected to an electronic device used for applying electric voltage;
a reactive pad disposed between the working electrode and the counter electrode and comprising an reactive reagent, wherein the reactive reagent comprises an electron transport material, an enzyme capable of reacting with the analyte and generating a current corresponding to the concentration, and a buffer solution; and
a separating pad disposed between the first and second substrate, wherein the blood sample is passed through the separating pad such that interfering materials including corpuscles can be separated and removed from the blood sample and then the blood sample is laterally diffused to the reactive pad
wherein the blood sample is a whole blood sample.

2. The sensing member of claim 1, wherein the substrates are made of a dielectric polymer.

3. The sensing member of claim 2 wherein the dielectric polymer is at least one of the group consisting of PVC, polystyrene, polyester, polycarbonate, polyether, polyethylene, polypropylene, and PET.

4. The sensing member of claim 1, wherein the substrates are made of one of the group consisting of SiO₂ and AlO₂.

5. The sensing member of any of the preceding claims, wherein the electrodes are made of one of the group consisting of carbon electrode, graphite electrode and metal particle.

6. The sensing member of any of the preceding claims, wherein the electrodes are formed on surfaces of the substrates through screen print.

7. The sensing member of any of the preceding claims, wherein the .electron transport material is selected from the group consisting of ferricyanate, ferrocene, para-benzoquinone, phenazine methosulfate, Indophenol, TMB(3,3',5,5'-Tetramethylbenzidine) and β-naphthoquinone-4-potassium sulfonate.

8. The sensing member of claim 7, wherein the ferricyanate is potassium ferricyanide.

9. The sensing member of any of the preceding claims, wherein the enzyme capable of reacting with the analyte and generating a current corresponding to the concentration comprises cholesterol esterase, cholesterol oxidase and catalase.

10. The sensing member of any of the preceding claims, wherein the buffer solution is one of the group consisting of phosphate buffer solution, TRIS buffer solution and MES buffer solution.

11. The sensing member of any of the preceding claims, wherein the reactive reagent further comprises a surface active reagent.

## Patentansprüche

1. Ein Meßfühler zum Erfassen des Gesamt-Cholesteringehalts einer Blutprobe, umfassend:
eine Arbeitselektrode und eine Gegenelektrode, welche jeweils auf einem ersten Substrat und einem zweiten Substrat ausgebildet sind;
eine dielektrische Schicht, welche auf einer der Oberflächen der ersten und dem zweiten Substrat ausgebildet ist und die Elektrode und einen Endverbinder freigelegt läßt, so daß der Endverbinder mit einer elektronischen Vorrichtung, welche zum Anlegen einer elektrischen Spannung verwendet wird, elektrisch verbunden werden kann;
ein reaktionsfähiges Kissen, welches zwischen der Arbeitselektrode und der Gegenelektrode angeordnet ist und ein Reaktionsmittel umfaßt, wobei das Reaktionsmittel ein Elektronentransportmaterial, ein Enzym, welches in der Lage ist, mit dem Analysematerial zu reagieren und einen Strom entsprechend der Konzentration zu erzeugen, und eine Pufferlösung umfaßt; und
ein Trennkissen, welches zwischen dem ersten und dem zweiten Substrat angeordnet ist, wobei die Blutprobe durch das Trennkissen geleitet wird, so daß störende Materialien, umfassend Korpuskeln, abgeschieden und aus der Blutprobe entfernt werden können, wobei die Blutprobe sodann seitwärts zu dem reaktionsfähigen Kissen geleitet wird,
wobei die Blutprobe eine Vollblutprobe ist.

2. Meßfühler nach Anspruch 1, wobei die Substrate aus einem dielektrischen Polymer hergestellt sind.

3. Meßfühler nach Anspruch 2, wobei das dielektrische Polymer mindestens eines aus der Gruppe ist, welche aus PVC, Polystyrol, Polyester, Polykarbonat, Polyether, Polyethylen, Polypropylen und PET besteht.

4. Meßfühler nach Anspruch 1, wobei die Substrate aus einem Vertreter der Gruppe, welche aus SiO₂ und AlO₂ besteht, hergestellt sind.

5. Meßfühler nach einem der vorangehenden Ansprüche, wobei die Elektroden aus einem Vertreter der Gruppe, welche aus Kohlenstoffelektroden, Graphitelektroden und Metallpartikeln besteht, hergestellt sind.

6. Meßfühler nach einem der vorangehenden Ansprüche, wobei die Elektroden durch Siebdruck auf Oberflächen der Substrate ausgebildet sind.

7. Meßfühler nach einem der vorangehenden Ansprüche, wobei das Elektronentransportmaterial aus der Gruppe, welche aus Ferricyanat, Ferrocen, para-Benzochinon, Phenazinmethosulfat, Indophenol, TMB(3,3',5,5'-Tetramethylbenzidin) und β-Naphthochinon-4-kaliumsulfonat besteht, ausgewählt ist.

8. Meßfühler nach Anspruch 7, wobei das Ferricyanat Kaliumhexacyanoferrat ist.

9. Meßfühler nach einem der vorangehenden Ansprüche, wobei das Enzym, welches in der Lage ist, mit dem Analysematerial zu reagieren und einen Strom entsprechend der Konzentration zu erzeugen, Cholesterinesterase, Cholesterinoxidase und Katalase umfasst.

10. Meßfühler nach einem der vorangehenden Ansprüche, wobei die Pufferlösung ein Vertreter aus der Gruppe ist, welche aus Phosphatpufferlösung, TRIS-Pufferlösung und MES-Pufferlösung besteht.

11. Meßfühler nach einem der vorangehenden Ansprüche, wobei das Reaktionsmittel ferner ein oberflächenaktives Reagens umfaßt.

## Revendications

1. Élément de détection destiné à déterminer la concentration de cholestérol total dans un échantillon de sang, comprenant :
une électrode active et une électrode de comptage conformées respectivement sur un premier substrat et un second substrat ;
une couche diélectrique conformée sur l'une des surfaces des premier et second substrats et exposant l'électrode et le connecteur terminal de telle manière que le connecteur terminal peut être électriquement connecté à un dispositif électronique utilisé pour appliquer une tension électrique ;
un tampon réactif disposé entre l'électrode active et l'électrode de comptage et comprenant un réactif, dans lequel le réactif comprend un matériau de transport d'électrons, une enzyme capable de réagir avec la substance analysée et produisant un courant électrique correspondant à la concentration, ainsi qu'une solution tampon ; et
un tampon de séparation disposé entre les premier et second substrats, dans lequel l'échantillon de sang passe à travers le tampon de séparation de telle manière que des substances perturbatrices comportant des particules puissent être séparées et éliminées de l'échantillon de sang et qu'ensuite l'échantillon de sang puisse diffuser latéralement vers le tampon réactif,
dans lequel l'échantillon de sang est un échantillon de sang total.

2. Élément de détection selon la revendication 1, dans lequel les substrats sont constitués d'un polymère diélectrique.

3. Élément de détection selon la revendication 2, dans lequel le polymère diélectrique comporte au moins un polymère choisi dans le groupe comprenant le PVC, le polystyrène, le polyester, le polycarbonate, le polyéther, le polyéthylène, le polypropylène et le PET.

4. Élément de détection selon la revendication 1, dans lequel les substrats sont constitués de SiO₂ ou de AlO₂.

5. Élément de détection selon l'une quelconque des revendications précédentes, dans lequel les électrodes sont des électrodes de carbone, de graphite ou de particules métalliques.

6. Élément de détection selon l'une quelconque des revendications précédentes, dans lequel les électrodes sont conformées sur les surfaces des substrats par sérigraphie.

7. Élément de détection selon l'une quelconque des revendications précédentes, dans lequel le matériau de transport d'électrons est choisi dans le groupe comprenant le ferricyanure, le ferrocène, la para-benzoquinone, le phénazine méthosulfate, l'indophénol, le TMB (3,3',5,5'-tétraméthylbenzidine) et le β-naphthoquinone-4-potassium sulfonate.

8. Élément de détection selon la revendication 7, dans lequel le ferricyanure est du ferricyanure de potassium.

9. Élément de détection selon l'une quelconque des revendications précédentes, dans lequel l'enzyme capable de réagir avec la substance analysée et de générer un courrant électrique correspondant à la concentration recherchée comprend de la cholestérol estérase, de la cholestérol oxydase et de la catalase.

10. Élément de détection selon l'une quelconque des revendications précédentes, dans lequel la solution tampon est une solution tampon de phosphate, une solution tampon TRIS ou une solution tampon MES.

11. Élément de détection selon l'une quelconque des revendications précédentes, dans lequel le réactif comporte en outre un réactif de surface.
